## Europäisches Patentamt

## European Patent Office

## Office européen des brevets

(11) Veröffentlichungsnummer: **0 172 130**
**B1**

(12)

# EUROPÄISCHE PATENTSCHRIFT

(45) Veröffentlichungstag der Patentschrift:
19.04.89

(51) Int. Cl.⁴: **A 61 B 17/58**

(21) Anmeldenummer: **85730086.7**

(22) Anmeldetag: **18.06.85**

(54) Schraube zur orthopädischen Fixation.

(30) Priorität: 15.06.84 DE 8418588 U
19.09.84 DE 3434807
05.06.85 EP 85730078

(43) Veröffentlichungstag der Anmeldung:
19.02.86 Patentblatt 86/8

(45) Bekanntmachung des Hinweises auf die Patenterteilung:
19.04.89 Patentblatt 89/16

(84) Benannte Vertragsstaaten:
AT CH DE FR GB IT LI SE

(56) Entgegenhaltungen:
FR-A-2 381 511
LU-A-59 229
US-A-4 242 932

ENGINEERING IN MEDICINE, Band 7, Nr. 2, April
1978, Seiten 114-123, HMSO, London, GB; B.A.
JORDAN et al.: "A review of the factors affecting
the design, specification and material selection of
screws for use in orthopaedic surgery"

(73) Patentinhaber: **MECRON MEDIZINISCHE
PRODUKTE GMBH, Nunsdorfer Ring 25- 27,
D-1000 Berlin 48 (DE)**

(72) Erfinder: **Anapliotis, Emmanuel, Tollensestrasse 16,
D-1000 Berlin 37 (DE)**

(74) Vertreter: **Christiansen, Henning, Dipl.- Ing.,
Patentanwalt CHRISTIANSEN Pacelliallee 43/45,
D-1000 Berlin 33 (DE)**

EP 0 172 130 B1

## Beschreibung

Die Erfindung betrifft eine Schraube der im Oberbegriff des Anspruchs 1 angegebenen Art. Eine Schraube dieser Gattung ist aus FR-A-2 381 511 bekannt.

Bei derartigen Knochenschrauben mit Innensechskant tritt vielfach das Problem auf, daß der Innensechskant der Knochenschraube sich im implantierten Zustand vom äußeren Rand her durch Kallusbildung zuzusetzen beginnt, so daß der Innensechskant für das zugehörige Drehwerkzeug infolge eines sich auftürmenden "Walls" unzugänglich wird. Daneben besteht die Gefahr, daß bei nicht vollständigem Einsetzen des Eindrehwerkzeuges bei den beim Ausdrehen der Schraube nach Verheilung des Knochens auftretenden Belastungen der Innensechskant beschädigt wird, so daß er sich verformt und gegebenenfalls nicht mehr die zur Übertragung des auftretenden Drehmoments notwendige Gestaltung aufweist und somit unbrauchbar wird.

Ferner dient bei Lochschrauben ein zuvor in den Knochen eingebrachter Führungsspieß, der auf das Innere der Knochenbohrung bezüglich des Durchmessers abgestimmt ist, zur Führung der Knochenschraube beim Einbringen in den Knochen.

Das zum Eindrehen erforderliche Drehmoment wird dabei ebenfalls über einen entsprechenden Mehrkantschraubendreher, der eine Durchgangsbohrung aufweist, mittels eines Innensechskants auf die Knochenschraube übertragen. Dieser Innensechskant weist wegen der Durchgangsbohrung größere Abmessungen auf als bei derartigen Schrauben üblicherweise verwendet werden bzw. welche für derartige Schrauben genormt sind.

Es kann daher gelegentlich vorkommen, daß zum Ausdrehen dieser Schrauben aus dem Knochen in einem Krankenhaus, welches normalerweise Schrauben ohne Loch verwendet, der Sechskant für Schlüssel mit Loch nicht im üblichen Instrumentarium vorhanden ist.

Der Erfindung liegt die Aufgabe zugrunde, bei Knochenschrauben der eingangs genannten Art eine größere Flexibilität hinsichtlich der Anschlußmöglichkeit für ein ein Eindrehmoment übertragendes Werkzeug zu schaffen, so daß bei Verformung der Anschlußöffnung bzw. Nichtvorhandensein eines vollständigen Satzes von Schraubwerkzeugen Ausweichmöglichkeiten bestehen.

Diese Aufgabe wird erfindungsgemäß mit einer Schraube, welche die Merkmale des kennzeichnenden Teils des Anspruchs 1 aufweist, gelöst.

Die Erfindung beruht insbesondere auf der Erkenntnis, daß zwei gleichartige Ausnehmungen mit abnehmendem Querschnitt - von außen nach innen hintereinander angeordnet - sich in das verrundete Unterteil einer Knochenschraube so einpassen lassen, daß auch in den tiefsten Bereichen der Ausnehmungen ein Restquerschnitt des Schraubenkopfes verbleibt, der eine den übrigen Momentübertragungsverhältnissen (Sechskante, Schraubenschaft) angepaßten Materialquerschnitt aufweist. Dadurch, daß die Belastbarkeit der einzelnen Sektionen der Schraube aufeinander abgestimmt ist, gestaltet sich der Gesamtentwurf insoweit ökonomisch, als kein Bereich wesentlich überdimensioniert zu sein braucht.

Die erfindungsgemäße Anordnung hat dabei weiterhin den Vorteil, daß durch das Vorsehen des kleineren Innensechskants im an den Schaft der Schraube angrenzenden Kopfbereich auch der Anschluß Kopf/Schaft einen ausreichenden Materialquerschnitt behält, welcher die sichere Überleitung der Drehmomente vom Kopf in den Schraubenschaft gewährleistet.

Dabei ist auch vorteilhaft, daß sich der - tiefer liegende - Sechskant mit kleinerem Querschitt auch dann noch problemlos benutzen läßt, wenn der äußere Sechskant in seinem der Öffnung zugewandten Bereich mit Kallusmaterial ausgefüllt ist. Infolge der stufenförmigen Ausbildung der Aufeinanderfolge der beiden Innensechskante wird in vorteilhafter Weise sichergestellt, daß trotz möglicherweise zugesetztem äußeren Randbereich ein dem kleineren Innensechskant angepaßtes Schraubwerkzeug ohne Schwierigkeiten einführbar ist.

Zusätzlich ergibt sich dabei der Vorteil, daß - falls der Randbereich des Innensechskants mit größerem Durchmesser ohne Schwierigkeiten zu reinigen ist - dieser zusätzlich wahlweise zum Ausdrehen der Schraube benutzt werden kann. (Zum Eindrehen stehen in jedem Fall beide Sechskantgrößen wahlweise zur Verfügung, so daß auf dasjenige Werkzeug zurückgegriffen werden kann, welches gerade zur Hand ist.)

In denjenigen Fällen, in denen ein besonders großes Ausdrehmoment erzeugt werden muß, steht gegebenenfalls ein Spezialwerkzeug zur Verfügung, welches einen gestuften Außensechskant aufweist, der an beide Innensechskante gleichzeitigermaßen angepaßt ist. Durch die einführbare Gesamtlänge des Sechskantes ergibt sich damit eine große Sicherheit gegen Verkanten, so daß der die Übertragung des größeren Drehmomentanteils gewährleistende Sechskant mit größerem Querschnitt zentriert und koaxial ausgerichtet ist, so daß ein das Ausbrechen von Kanten bewirkendes Verkanten des Außensechskantes beim Einführen in den Innensechskant verhindert ist.

Der erfindungsgemäße Doppelinnensechskant ist im Zusammenwirken mit einer durchbohrten Schraube und der Anwendung mit einem Führungsspieß besonders vorteilhaft. Beim Einschrauben wird dabei ein Führungsdraht verwendet, der die fluchtende Ausrichtung von Bohrloch, Schraube und (ebenfalls durchbohrtem) Schlüssel bewirkt. Beim Ausschrauben wird bevorzugt ein Schlüssel mit dem kleineren Innensechskant benutzt, wobei in der Anwendung mit Knochenschrauben und voll eingedrehter Schraube das Fluchten von Schraubenlängsachse und Schlüssel an und für sich nur schwer zu übersehen ist. Ein Verkanten des Sechskantschlüssels führt aber am ehesten zu einer Deformation des kleineren Innensechskants. Bei dem erfindungsgemäßen Doppelsechskant ist die Ausrichtung des kleineren Sechskantschlüssels durch Beobachtung von dessen Position in bezug auf den großen Innensechskant jedoch jederzeit gut zu kontrollieren. Beim Blick von oben auf die Schraube wird der'das Instrument benutzende Chirurg von sich aus ohne besondere diesbezügliche Anweisung darauf achten, daß das den Sechskant des Schlüssels umgebende Schattenfeld an allen Kantenflächen gleiche Breite aufweist.

Die beiden Sechskante sind bevorzugt so ausgelegt, daß sie in etwa gleich große Momente aufnehmen können, bis sie durch Verformung unbrauchbar werden, so daß ein Ausdrehen der Schraube mit dem kleineren Innensechskant (unter Zugrundelegung übereinstimmender Momente für das Ein- bzw. Ausdrehen) in jedem Falle noch gesichert ist.

Die erfindungsgemäße Schraube ist weiterhin in günstiger Weise insgesamt derart ausgebildet, daß nicht nur die durch die beiden Innensechskante, sondern auch die durch den verbleibenden Querschnitt des Kopfes übertragbaren Momente in etwa gleich groß sind, so daß sichergestellt ist, daß auch der Kopf beim Aufbringen der maximalen Ein- bzw. Ausdrehmomente nicht abgedreht wird. Dabei erweist es sich als besonders günstig, wenn der untere Bereich des Kopfes im Bereich der gestuft angeordneten Innensechskante sich verjüngend ausgebildet ist, so daß die Außenkontur des Kopfes in etwa der Verjüngung der inneren Ausnehmung folgt und somit der verbleibende Restquerschnitt des Materials im Minimum in etwa gleich bleibt.

Ist der kleinere Außensechskant geringfügig länger als der größere Innensechskant der Schraube, so ist bei einem entsprechenden Schraubendreher mit gestuftem Sechskant zudem gewährleistet, daß der kleinere Sechskant zuerst faßt und den großen Sechskant beim weiteren Einführen des Werkzeugs zentriert, so daß ein schädliches verkantetes Einführen bei behindernder Materialanlagerung im größeren Innensechskant verhindert ist.

Zur Anwendung für Knochenschrauben mit einem sich über die gesamte Schaftlänge erstreckenden Gewinde mit mindestens zum Teil selbstschneidender Wirkung kann (wegen der auftretenden unterschiedlichen Momente) beim Ausdrehen aus dem Knochen ein Sechskant mit kleinerer Festigkeit bezüglich der das Drehmoment übertragenden Querschnitts verwendet werden als beim Eindrehen. Bei Lochschrauben kann der Querschnitt des Werkzeugs zum Ausschrauben auch deswegen kleiner bemessen sein, weil die Bohrung für den Führungsspieß der Lochschraube enfällt. Die zusätzlichen die Übertragung eines Drehmoments ermöglichen den Anschlußflächen des weiteren Sechskants können in diesem Fall insgesamt kleiner gehalten werden als der Innensechskant zur Aufnahme eines Eindrehschlüssels mit Ausnehmung für einen inneren Führungsspieß.

Bei einer Ausführung der Knochenschraube, welche nicht über ihre volle Schaftlänge mit einem Gewinde versehen ist, muß sich diese beim Entfernen rückwärts erneut ein Gewinde schneiden, da das ursprünglich zum Einfügen der Schraube vorgeschnittene Gewinde mit Knochensubstanz zugewachsen ist. In diesem Fall ist es günstig, wenn das Gewinde der Schraube in seinem rückwärtigen Bereich Schneidnuten aufweist, so daß die anfallenden Schneidspäne aufgenommen werden können und somit das Herausdrehen der Schraube nicht behindern.

Bei einer anderen günstigen entsprechenden Ausführung der Schraube ist das dem Kopf zugewandte Ende des Gewindes "selbstfurchend" ausgebildet, wobei es sich über mindestens einen Gewindegang bis auf den Schaftquerschnitt verjüngt, so daß es sich beim Ausdrehen der Schraube nach dem Einheilen in den Knochen ein neues Gewinde eingräbt, wobei der - in Schraubrichtung - stetig zunehmende Außendurchmesser die mit hartem, neu gebildeten Kallusmaterial ausgefüllten ehemaligen Gewindegänge verdrängt ohne, daß eine wesentliche Erhöhung des Ausschraubmoments resultiert. Zur Verkleinererung dieses Moments trägt es auch bei, wenn der nicht mit einem Gewinde versehene Schaftquerschnitt in bevorzugter Weise gegenüber dem Innendurchmesser des Gewindes vergrößert ist, so daß die Flanken sich höchstens mit ihren Spitzen einschneiden müssen.

Andere vorteilhafte Weiterbildungen der Erfindung sind in den Unteransprüchen gekennzeichnet bzw. werden nachstehend anhand einer bevorzugten Ausführung näher beschrieben. Es zeigen:

Figur 1 zeigt ein Ausführungsbeispiel der erfindungsgemäßen Schraube im Querschnitt,

Figuren 2 und 3 jeweils einen Schraubendreher mit einfachem und doppeltem Sechskant in Teildarstellungen,

Figuren 4 bis 6 Einzelheiten eines weiteren Ausführungsbeispiels einer Schraube gemäß der Erfindung sowie

Figuren 7a bis c ein Detail einer weiteren Ausführungsform in verschiedenen Positionen beim Ausschrauben.

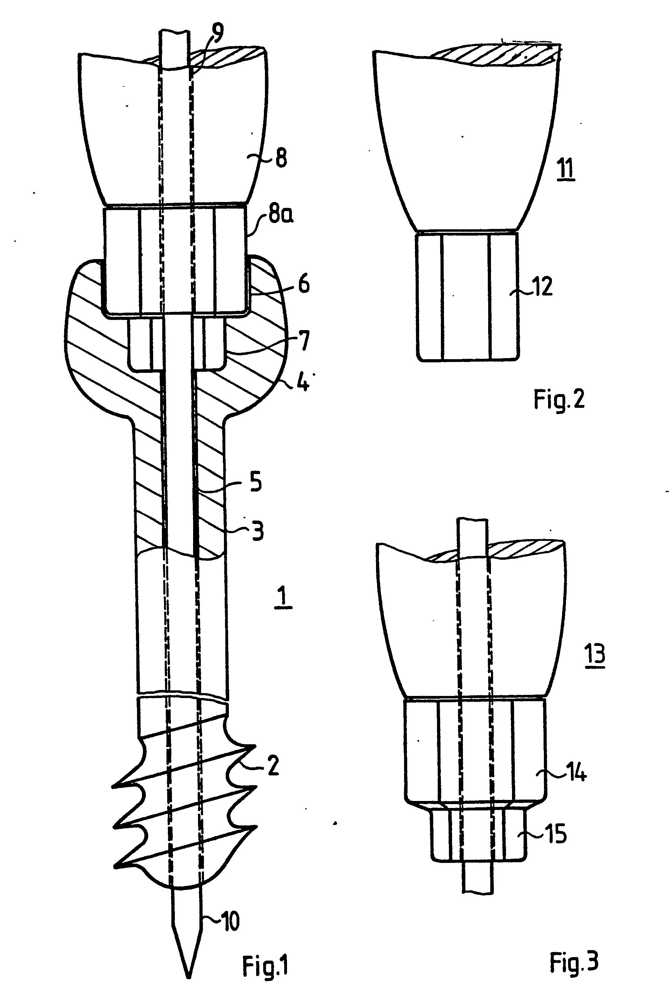

Bei dem in der Figur 1 dargestellten Ausführungsbeispiel ist eine Knochenschraube 1 im Schnitt dargestellt, welche mit einem Gewinde 2 versehen ist, das als Spongiosagewinde mit besonderer Gestaltung der Flanken für Knochenschrauben Verwendung findet. Die Schraube weist einen Schaftbereich 3 ohne Gewinde und einen Kopf 4 auf, der nach außen hin verrundet und bezüglich seiner Unterseite an die Gestaltung von Knochenplatten etc. angepaßt ist. Die Schraube 1 enthält eine durchgehende Bohrung 5, die bei dem dargestellten Ausführungsbeispiel einen Durchmesser von 1,5 mm besitzt.

Der Innenquerschnitt des Kopfes ist mit zwei sechseckigen Ausnehmungen 6 und 7 versehen, wobei der an die Außenoberfläche anschließende Innensechskant 6 größer ausgebildet ist als der Innensechskant 7, welcher sich zum Inneren hin koaxial an den Sechskant 6 anschließt. Während der Innensechskant 6 einen Durchmesser von 8 mm (entsprechend einer Schlüsselweite SW 5) aufweist, ist der Durchmesser des Innensechskants 7 nur 5 mm, entsprechend einer Schlüsselweite SW 3,5. Die Höhe der beiden Sechskante ist in etwa übereinstimmend gewählt, wobei die Summe der Höhen geringfügig geringer als die Höhe des Kopfes 4 der Schraube ist. Die beiden Innensechskante 6 und 7 und die Bohrung 5 sind koaxial in bezug auf den Schaft 3 der Schraube 1 ausgerichtet.

In Figur 1 ist in den größeren Innensechskant der Außensechskant eines Schlüssels 8 mit einem Außensechskant 8a eingesteckt, welcher ebenfalls mit einer Innenbohrung 9 versehen ist, durch den ein Führungsspieß 10 geführt wird, welcher in Längsrichtung das Innere der Schraube 1 durchquert und die Knochenschraube beim Eindrehen zentriert.

Der Außensechskant 8a des Eindrehwerkzeugs ist so groß bemessen, daß er trotz Schwächung durch die Innenbohrung noch eine genügende Stabilität aufweist, um das zum Eindrehen der Schraube in den Knochen aufzubringende Drehmoment zu übertragen.

Soll nun die dargestellte Schraube aus dem Knochen entfernt werden, so kann hilfsweise der Innensechskant 7 verwendet werden, für den ein entsprechend angepaßter Sechskantschlüssel 11 in Figur 2 wiedergegeben ist. Dieser Schlüssel weist einen Außensechskant 12 auf, welcher an den Innensechskant 7 der Schraube 1 angepaßt und zur Übertragung der zum Ausdrehen der Schraube aufzubringenden Drehmomente geeignet ist.

Der Innensechskant 7 entspricht in seinen Abmessungen den für die zugehörigen Schraubengrößen üblicherweise verwendeten Sechskanten, wobei bei der dargestellten Ausführung der Schraube infolge der Anbringung zweier Innensechskante eine Verkürzung in Längsrichtung im Vergleich zu den üblichen Sechskanten vorgenommen ist. Diese Verkürzung ist deswegen weitgehend ohne Bedeutung, weil die Drehmomentübertragung vorzugsweise zum Ausdrehen für Anwendungsfälle vorgesehen ist, bei denen zum Herausschrauben ein kleineres Moment notwendig ist. Der zum Eindrehen der Schraube benutzte Innensechskant 6 ist zwar ebenfalls in bezug auf die Länge des Schraubenkopfes 4 verkürzt, da er aber gegenüber der üblichen Abmessung einen vergrößerten Querschnitt aufweist, kann er trotz seiner verkürzten Form die zum Einschrauben aufzubringenden Drehmomente sicher aufnehmen. Insoweit zeigt sich also, daß die Lösung in der dargestellten Ausführungsform mit den bestehenden Anforderungen vereinbar ist. Werden zum Ein- oder Ausdrehen der Schraube 1 beide Innensechskante gleichzeitig herangezogen, wie es mittels des in Figur 3 dargestellten Werkzeugs 13 mit doppeltem Sechskantanschluß möglich ist, läßt sich das insgesamt übertragbare Drehmoment sogar noch steigern, da der Kopfraum der Schraube gegenüber einer herkömmlichen Schraube vergrößerte Flächen zur Drehmomentübertragung enthält. Der Materialquerschnitt des restlichen Kopfes zur Übertragung der Drehmomente in den Schraubenschaft ist dagegen kaum vermindert und entspricht wegen der verrundeten Form der Kopfunterseite an jeder Stelle den durch die Eindrehwerkzeuge und die Sechskante übertragbaren Momenten.

In Figur 3 ist - entsprechend - Figur 2 ein Teil eines Werkzeugs 13 wiedergegeben, welches zwei koaxial aneinander anschließende Innensechskante 14 und 15 aufweist, wobei beide Sechskante an die Abmessungen der beiden Innensechskante der Schraube gemäß Figur 1 angepaßt sind.

Dieses Werkzeug gestattet somit eine maximale Drehmomentübertragung, wenn es in den Kopf der Schraube gemäß Figur 1 eingesetzt ist. Der kleinere Außensechskant 15 ist geringfügig länger als der größere Innensechskant der Schraube 1, so daß der kleinere Sechskant beim Einsetzen des Werkzeugs 13 zuerst faßt und den großen Sechskant beim weiteren Einführen des Werkzeugs zentriert, so daß ein schädliches verkantetes Einführen bei behindernder Materialanlagerung im größeren Innensechskant der Schraube verhindert ist. Wie in Figur 3 angedeutet, ist das Werkzeug 13 auch zum Eindrehen von Lochschrauben unter Benutzung eines Führungsspießes verwendbar.

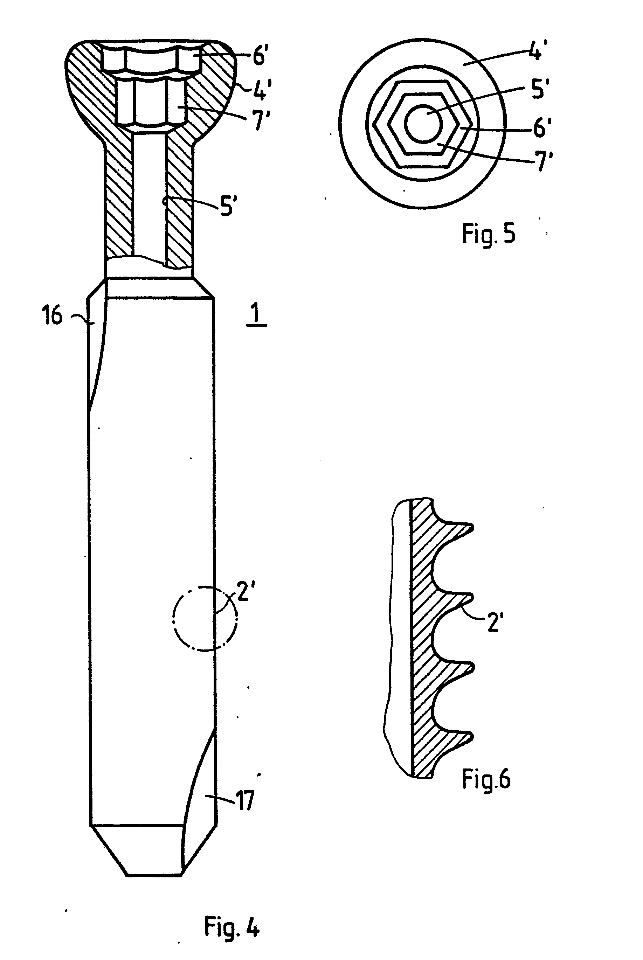

In den Figuren 4 bis 6 ist ein weiteres Ausführungsbeispiel der erfindungsgemäßen Schraube wiedergegeben, wobei die Elemente, welche denjenigen beim zuvor dargestellten Ausführungsbeispiel entsprechen mit einem "'" versehen sind. Es ist ersichtlich, daß der kleinere Innensechskant 7' tiefer ausgeführt ist als der Innensechskant 6' mit größerem Durchmesser. Auf diese Weise sind die maximalen übertragbaren Momente in etwa gleich, so daß die Schraube mit dem größeren Innensechskant nur insoweit eingeschraubt werden kann, als sie auch mit dem kleinen Innensechskant wieder entfernbar ist.

In der Draufsicht gemäß Figur 5 ist die konzentrische Anordnung der Sechskante 6' und 7' mit der Innenbohrung 5' erkennbar.

Um das Ausdrehen der Schraube auch dann ohne größeren Mehraufwand an Drehmoment zu ermöglichen, ist das Knochenschraubengewinde 2', welches als Einzelheit im Schnitt in Figur 6 separat dargestellt ist, mit einer Schneidnut 16 bzw. 17 nicht nur am vorderen, sondern auch am rückwärtigen Ende des Gewindes versehen. Auf diese Weise können Schneidspäne aufgenommen werden und führen nicht zu einem Verklemmen. Die Schneidnut 16 bzw. 17 besteht aus einem längsgerichteten Schlitz, welcher das Gewinde durchquert und vom Ende des Gewindes her gesehen an Tiefe abnimmt bis er nach einiger Zeit ausläuft. Die Zahl der durchquerten Gewindegänge beträgt bei einem bevorzugten Ausführungsbeispiel ungefähr zehn. Eine derartige Schneidnut an dem dem Schraubenkopf benachbarten Gewindeende ist für alle Knochenschrauben von Bedeutung, die entfernt werden müssen, nachdem sich das beim Einschrauben vorgeschnittene Gewinde wieder zum Teil mit Knochenmaterial gefüllt hat.

Der Kopf der Schraube 4' ist an seiner Unterseite verrundet, so daß der Schraubenkopf in einer balligen Ausnehmung in unterschiedlichen Richtungen anliegen kann. Der sich derart verjüngende Kopf folgt der gestuften Querschnittsverringerung der Innensechskante, so daß der verbleibende tragende Querschnitt des Kopfes im Mittel konstant bleibt und die Tragfähigkeit auch nicht diejenige des Schraubenschaftes unterschreitet. Der im Bereich des Endes des kleineren Innensechskantes 7' verbleibende Querschnitt entspricht im wesentlichen demjenigen des Schaftes der Hohlschraube.

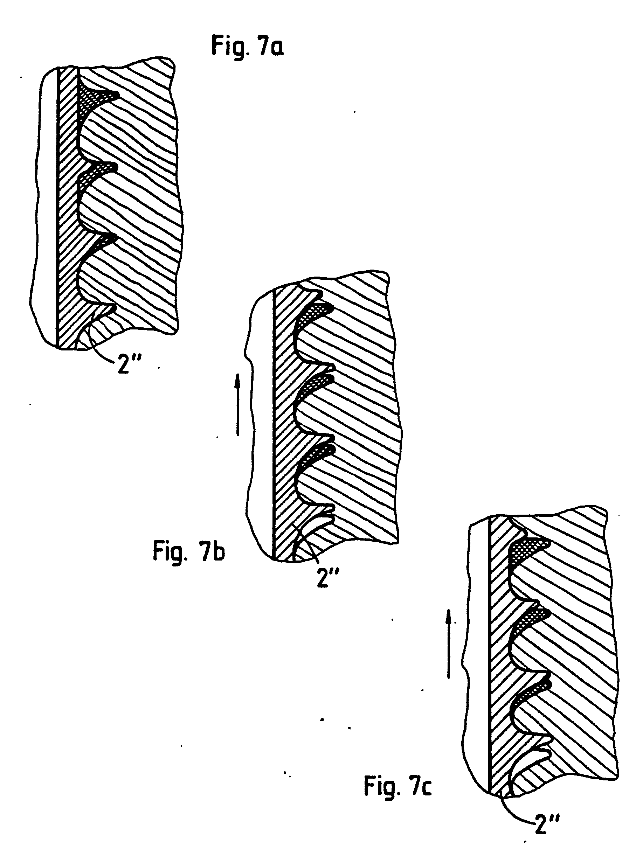

In den Figuren 7a bis 7c ist die Wirkung einer in Rückwärtsrichtung mit einem selbstschneidenden im Sinne eines selbstfurchenden Gewindes 2'' ausgestatteten Knochenschraube dargestellt. Die selbstfurchende Ausbildung der Schraube besteht darin, daß das Knochenschraubengewinde über mindestens einen - vorzugsweise mehrere - Gewindegänge in seinem Außenumfang abnimmt, wobei der Kerndurchmesser erhalten bleibt und sich die Verringerung des Querschnitts des die Flanken tragenden Bereichs des Gewindes bevorzugt auf den Teil erstreckt der eine stärkere Neigung in bezug auf die radiale Richtung aufweist. Bei Knochenschraubengewinden verläuft bekanntlich eine Flanke des Gewindes im wesentlichen in einer radial gerichteten Ebene, da dieser Teil in Zugrichtung der Schraube möglichst große Kräfte auf den Knochen übertragen soll.

In den Figuren 7a bis 7c ist ersichtlich, wie der rückwärtige Teil des Gewindes beim Ausschrauben sich erneut in das Knochenmaterial einschneidet, wobei die Gewindegänge mehr und mehr benachbart zu den beim Einschrauben erzeugten Gewindegängen eingefurcht werden, welche sich während des Zeitraums der Verheilung mit hartem Kalusmaterial gefüllt haben.

In den Figuren 7a ist der rückwärtige Gewindebereich der erfindungsgemäßen Knochenschraube dargestellt, wie er sich nach einiger Zeit darstellt, wenn die beim Einschrauben erzeugten Gewindegänge wieder mit Knochmaterial angefüllt sind. Es ist ersichtlich, daß das Gewinde im Endbereich von unten nach oben bezüglich der Flanken in seinen Durchmesser verringert ist, während der Kerndurchmesser erhalten bleibt.

In Figur 7b ist die Schraube um einen Gewindegang zurückgedreht dargestellt, wobei sich der auslaufende Teil des Gewindes mit einer Tendenz nach oberhalb des mit Knochenmaterial zugewachsenen verhärteten alten Gewindegangs einen neuen Gewindegang erzeugt. Die nachfolgenden im Durchmesser zunehmenden Teile des Gewindes erweitern die vorhandenen Gänge ebenfalls in Richtung zum Ausgang des Gewindes hin, wobei sich die Gewindeflanken auf dem verhärteten Knochenmaterial abstützen, so daß diese sozusagen ein neues vorgefertigtes Innengewinde bildet, wobei das Außengewinde der Schraube sich zwischen dem verhärteten Gewindegängen eingräbt. Zum Teil wird das verhärtete alte Gewinde auch axial verdrängt. Dieser Vorgang ist noch deutlicher in Figur 7c zu sehen, wo bereits ein Ausdrehen um zwei Gewindegänge (zwei Umdrehungen) vorgenommen wurde. Auf diese Weise wird ersichtlicherweise das Ausdrehen der Knochenschraube erleichtert. Weiter begünstigt wird dieses Ausdrehen noch, wenn der gewindelose Schaftquerschnitt einen gegenüber dem Innendurchmesser des Gewindes vergrößerten Durchmesser aufweist, so daß sich das Gewinde beim Herausschrauben nur in verringerter Tiefe einschneiden muß.

**Patentansprüche**

1. Schraube zur orthopädischen Fixation, insbesondere mit einer in Längsrichtung durchgehenden Bohrung, mit einem konzentrischen Innensechskant im Kopfbereich, dadurch gekennzeichnet, daß innerhalb des Kopfes (4, 4') eine weiterer Innensechskant (7, 7') vorgesehen ist, welcher kleiner als der erste Sechskant (6, 6') ausgebildet ist und sich in koaxialer Ausrichtung an den ersten Innensechskant in Längsrichtung zum Schraubeninneren hin anschließt.

2. Schraube nach Anspruch 1, dadurch gekennzeichnet, daß die beiden Innensechskante (6, 6' und 7, 7') bezüglich ihrer Innenflächen parallel ausgerichtet sind.

3. Schraube nach einem der vorangehenden Ansprüche, dadurch gekennzeichnet, daß beide Innensechskante (6' und 7') innerhalb des Kopfes (4') so angeordnet sind, daß die verrundete Schraubenunterseite der Kontur der Sechskante derart folgt, daß die durch die Sechskante geschwächten Querschnitte im Bereich von deren unteren Kanten eine im wesentlichen gleiche Belastbarkeit aufweisen, welche im wesentlichen auch der Belastbarkeit des Schaftquerschnitts nahe dem Kopf entspricht.

4. Schraube nach einem der vorangehenden Ansprüche, dadurch gekennzeichnet, daß sich die Querdurchmesser der Innensechskante (6 und 7) im wesentlichen wie 8 zu 5 verhalten.

5. Schraube nach einem der vorangehenden Ansprüche, dadurch gekennzeichnet, daß die Querdurchmesser/Schlüsselweite der Innensechskante (6, 6' und 7, 7') 3,5 mm und 2,5 mm bzw. 5,0 mm und 3,5 mm beträgt.

6. Schraube nach einem der vorangehenden Ansprüche, dadurch gekennzeichnet, daß die Tiefe der beiden Sechskante (6' und 7') derart gewählt ist, daß sie für sich eine im wesentlichen gleiche Belastbarkeit aufweisen.

7. Schraube nach einem der vorangehenden Ansprüche, welche ein Gewinde (2') aufweist, das sich nicht über die volle Länge des Schaftes erstreckt, dadurch gekennzeichnet, daß mindestens an einem Ende des Gewindes eine Schneidnut (16) vorgesehen ist.

8. Schraube nach Anspruch 7, dadurch gekennzeichnet, daß die Tiefe der Schneidnut (16, 17) sich vom Ende des Gewindes her zunehmend vermindert.

9. Schraube nach einem der vorangehenden Ansprüche, dadurch gekennzeichnet, daß das das rückwärtige Ende der Schraube in der Weise selbstschneidend - d. h. selbstfurchend - ausgebildet ist, daß sich der äußere Durchmesser des dem Kopf zugewandten Endes des Gewindes über mindestens einen Gang im wesentlichen bis auf den Schaftdurchmesser verringert.

10. Schraube nach einem der vorangehenden Ansprüche, dadurch gekennzeichnet, daß eine in Längsrichtung durchgehende Innenbohrung (5, 5') vorgesehen ist.

## Claims

1. Screw for orthopaedic fixing, in particular with a bore running through longitudinally, with a concentric inner hexagon in the region of the head,
characterized in that
inside the head (4, 4') another inner hexagon (7, 7') is provided, which is smaller than the first hexagon (6, 6') and which attaches coaxially, and longitudinally with respect to the interior of the screw, to the first inner hexagon.

2. Screw according to claim 1, characterised in that the two inner hexagons (6, 6' and 7, 7') are parallel in respect of their inner surfaces.

3. Screw according to any one of the foregoing claims, characterized in that both inner hexagons (6' and 7') are so arranged within the head (4') that the rounded underside of the screw follows the contour of the hexagon in such a manner that the cross-sections weakened by the hexagon in the region of the hexagon's lower edges are of substantially equal loading capacity, which also substantially corresponds to the loading capacity of the cross-section of the shaft near the head.

4. Screw according to any one of the foregoing claims, characterized in that the transverse diameters of the inner hexagons (6 and 7) are essentially in the ratio of 8 to 5.

5. Screw according to any one of the foregoing claims, characterized in that the transverse diameter/width across flats of the inner hexagon (6, 6' and 7, 7') is 3.5 mm and 2.5 mm, and 5.0 mm and 3.5 mm respectively.

6. Screw according to any one of the foregoing claims, characterized in that the depth of the two hexagons (6' and 7') is chosen so that they are of substantially equal loading capacity.

7. Screw according to any one of the foregoing claims, which has a thread (2') which does not extend over the full length of the shaft, characterized in that a cutting groove (16) is provided at least at one end of the thread.

8. Screw according to claim 7, characterised in that the depth of the cutting groove (16, 17) diminishes increasingly from the end of the thread onwards.

9. Screw according to any one of the foregoing claims, characterized in that the tail end of the screw is designed to be self-cutting - i.e. self-grooving - , so that the exterior diameter of the end of the thread facing the head diminishes over at least one thread substantially right up to the shaft diameter.

10. Screw according to any one of the foregoing claims, characterized in that an inner bore (5, 5') is provided, running through longitudinally.

## Revendications

1. Vis pour fixation orthopédique, en particulier avec un perçage traversant dans la direction longitudinale, avec un six pans creux concentrique dans la zone de tête,
caractérisée
en ce qu'à l'intérieur de la tête (4, 4') est prévu un autre six pans creux) (7, 7') qui est conçu plus petit que le premier six pans creux (6, 6') et qui se raccorde, en alignement coaxial, au premier six pans creux dans la direction longitudinale, en allant vers l'intérieur de la vis.

2. Vis selon la revendication 1, caractérisée en ce que les deux six pans creux (6, 6' et 7, 7') sont orientés parallèlement en ce qui concerne leurs faces intérieures.

3. Vis selon l'une des revendications précédentes, caractérisée en ce que deux six pans creux (6' et 7') sont disposés à l'intérieur de la tête (4') de façon telle que la face inférieure, arrondie, de la vis suit le contour des six pans creux de façon telle que les sections affaiblies par les six pans creux présentent, dans la zone de leurs arêtes inférieures, une capacité de charge sensiblement identique, qui correspond sensiblement aussi à la capacité de charge de la section du fût près de la tête.

4. Vis selon l'une des revendications précédentes, caractérisée en ce que les diamètres transversaux des six pans creux (6 et 7) sont sensiblement dans le rapport de 8 à 5.

5. Vis selon l'une des revendications précédentes, caractérisée en ce que le diamètre transversaux/la dimension nominale de clé des six pans creux (6, 6' et 7, 7') valent 3,5 mm et 2,5 mm ou 5,0 mm et 3,5 mm.

6. Vis selon l'une des revendications précédentes, caractérisée en ce que l'on choisit la profondeur des deux six pans creux (6' et 7') de façon telle qu'ils présentent en soi une capacité de charge sensiblement égale.

7. Vis selon l'une des revendications précédentes, qui présente un filetage (2') qui ne s'étend pas sur toute la longueur du fût, caractérisée en ce qu'au moins à l'une des extrémités du filetage est prévue une rainure de coupe (16).

8. Vis selon la revendication 7, caractérisée en ce que la profondeur de la rainure de coupe (16, 17) décroît progressivement depuis l'extrémité du filetage.

9. Vis selon l'une des revendications précédentes, caractérisée en ce que l'extrémité arrière de la vis est conçue autocoupante -c'st-à-dire autotaraudeuse - de façon telle que le diamètre extérieur de l'extrémité, côté tête, du filetage diminue sensiblement jusqu'à arriver au diamètre du fût sur au moins un filet.

10. Vis selon l'une des revendications précédentes, caractérisée en ce qu'il est prévu un perçage intérieur traversant (5, 5') dans la direction longitidunale.

Fig.1

Fig.2

Fig.3

Fig. 4

Fig. 5

Fig.6

Fig. 7a

2″

Fig. 7b

2″

Fig. 7c

2″